# FASCICULE DE BREVET EUROPEEN

(11) **EP 1 848 794 B1**
(45) Date de publication et mention de la délivrance du brevet: **13.06.2012**
(21) Numéro de dépôt: 06709247.8
(22) Date de dépôt: 06.02.2006
(51) Int. Cl.: C12N 1/04, A61K 9/42, A61K 39/00

(54) **COMPOSITIONS COMPRENANT DES MICRO-ORGANISMES DESHYDRATES, LEURS PROCEDES DE PREPARATION, ET LEURS UTILISATIONS**
DEHYDRATISIERTE MIKROORGANISMEN UMFASSENDE ZUSAMMENSETZUNGEN, VERFAHREN ZUR HERSTELLUNG DAVON UND VERWENDUNGEN DAVON
COMPOSITIONS COMPRISING DEHYDRATED MICRO-ORGANISMS, METHOD FOR PREPARING SAME, AND USES THEREOF

(30) Priorité: 07.02.2005 FR 0501232
(43) Date de publication de la demande: 31.10.2007
(73) Titulaire: VIRBAC S.A., F-06516 Carros (FR)
(72) Inventeur: DERRIEU, Guy, 06800 Cagnes sur Mer (FR)
(74) Mandataire: Macquet, Christophe
(86) Numéro de dépôt international: PCT/FR2006/000258
(87) Numéro de publication internationale: WO 2006/082328

(56) Documents cités:
- EP-A- 0 486 234
- FR-A- 2 806 417
- US-A1- 2004 175 389

## Description

La présente invention concerne des compositions comprenant des micro-organismes déshydratés revivifiables, des procédés de préparation de telles compositions, et leurs utilisations en tant qu'apport alimentaire, notamment diététique, ou en tant que médicament.

La flore intestinale de l'Homme et des animaux, en particulier des animaux à sang chaud, présente de nombreuses espèces de micro-organismes. Il s'agit notamment de bactéries dont la présence est essentielle, non seulement à la digestion, mais aussi, et plus généralement, à la santé.

Aussi, de nombreux produits alimentaires ou médicaments ont été développés en vue de fournir des micro-organismes susceptibles de reconstituer la flore intestinale de l'Homme et des animaux.

Pour la fabrication de tels produits alimentaires ou de tels médicaments, les micro-organismes sont généralement préalablement déshydratés. Ils sont en outre associés à des composés, lesdits composés ayant avantageusement des propriétés prébiotiques, c'est-à-dire étant susceptibles de stimuler la croissance des micro-organismes.

Toutefois, les micro-organismes déshydratés de ces produits alimentaires sont soumis à des stress. Il s'agit de stress dus à l'environnement. On citera par exemple la chaleur et l'humidité. Il s'agit en outre de stress qui ne sont pas nécessairement dus à l'environnement. On citera par exemple les stress chimiques, tels que l'acidité, ou les stress enzymatiques.

Ces stress ont pour effet d'affecter la viabilité des micro-organismes déshydratés de sorte qu'en pratique, il n'est pas possible de garantir la présence d'une quantité substantielle de micro-organismes revivifiables dans ces produits alimentaires et ce, même lorsque le temps de conservation desdits produits est bref.

Bien sûr, on a cherché à développer des compositions comportant des micro-organismes déshydratés revivifiables dans lesquelles lesdits micro-organismes sont protégés des stress physico-chimiques auxquels ils sont normalement soumis.

Ces compositions visent à protéger les micro-organismes en les enrobant d'une substance protectrice. Elles sont notamment divulguées dans les documents publiés sous les numéros FR-2 806 417, FR-2 748 752 et US-2004/0175389. Dans le document FR-2 806 417, des micro-organismes sont enrobées par une substance protectrice hydrophobe. Par ailleurs, dans le brevet FR-2 748 752, des bactéries sont enrobées par des polymères. Enfin, dans le document US-2004/0175389, des bactéries sont enrobées par un gel d'acide alginique.

Néanmoins, l'enrobage de bactéries déshydratées est une opération qui, en elle-même, est stressante et qui, par suite, aboutit à une destruction d'une quantité substantielle des bactéries présentes dans les compositions obtenues. En outre, l'enrobage est une opération relativement complexe et aléatoire au fil des productions.

Compte tenu de ce qui précède, un problème que se propose de résoudre l'invention est de réaliser une composition comprenant des micro-organismes déshydratés revivifiables, ainsi qu'un procédé de préparation d'une telle composition, qui présente une certaine stabilité des micro-organismes dans le temps.

La solution de l'invention à ce problème posé a pour premier objet une composition comprenant des micro-organismes déshydratés revivifiables, caractérisée en ce qu'elle comprend en outre des particules dont au moins 50% ont un diamètre moyen supérieur à 250 µm.

Elle a pour second objet un procédé de préparation d'une composition alimentaire ou médicamenteuse, caractérisé en ce qu'elle comporte les étapes suivantes de : - déshydratation de micro-organismes revivifiables; et de - mélange des micro-organismes déshydratés avec des particules dont au moins 50% ont un diamètre moyen supérieur à 250 µm.

Enfin, elle a pour troisième objet l'utilisation d'une composition telle que ci-dessus, comme apport alimentaire ou comme médicament à usage humain ou vétérinaire.

De manière surprenante, lorsqu'une composition comprend à la fois des micro-organismes déshydratés et des particules dont au moins 50% ont un diamètre moyen supérieur à 250 µm, une quantité substantielle de micro-organismes déshydratés, en pratique plus de 20 %, reste revivifiable pendant un minimum de 18 mois.

L'invention sera mieux comprise à la lecture de la description non limitative qui va suivre.

La composition selon l'invention comprend des micro-organismes et des particules.

Les micro-organismes de la composition sont des organismes unicellulaires eucaryotes ou procaryotes de taille microscopique. Il s'agit préférentiellement de bactéries ou de levures et, plus préférentiellement, de bactéries ou levures présentant des propriétés probiotiques, c'est-à-dire utiles à l'organisme du corps humain ou animal.

Parmi les bactéries susceptibles d'entrer dans les compositions selon l'invention, on choisira avantageusement les bactéries de la famille des *Lactobacillaceae,* des *Bifidobacteriaceae,* des *Streptococcaceae,* des *Enterococcaceae* ou des *Bacillaceae.* A titre d'exemples non limitatifs de bactéries de la famille des *Lactobacillaceae,* on peut citer les espèces suivantes : *Lactobacillus acidophilus, Lactobacillus amylovorus, Lactobacillus brevis, Lactobacillus bulgaricus, Lactobacillus casei, Lactobacillus casei rhamnosus, Lactobacillus delbrueckii, Lactobacillus farciminis, Lactobacillus fermentum, Lactobacillus gasseri, Lactobacillus helveticus, Lactobacillus johnsonii, Lactobacillus lactis, Lactobacillus paracasei, Lactobacillus plantarum, Lactobacillus pentosaceus, Lactobacillus reuteri, Lactobacillus rhamnosus, Lactobacillus salivarius et Pediococcus acidilactici.* A titre d'exemples non limitatifs de bactéries de la famille des *Bifidobacteriaceae,* on peut citer les espèces suivantes . *Bifidobacterium animalis, Bifidobacterium bifidum, Bifidobacterium breve, Bifidobacterium infantis, Bifidobacterium lactis, *Bifidobacterium longum* et *Bifidobacterium pseudolongum.* A titre d'exemples non limitatifs de bactéries de la famille des *Streptococcaceae,* on peut citer les espèces suivantes : *Streptococcus infantarius, Streptococcus thermophilus, Streptococcus salivarius, et Lactococcus lactis.* A titre d'exemples non limitatifs de bactéries de la famille des *Enterococcaceae*, on peut citer l'espèce *Enterococcus faecium.* A titre d'exemples non limitatifs de bactéries de la famille des *Bacillaceae*, on peut citer les espèces suivantes : *Bacillus cereus, Bacillus licheniformis, Bacillus subtilis.*

Parmi les levures susceptibles d'entrer dans les compositions selon l'invention, on choisira avantageusement les levures de la famille des *Saccharomycetaceae*. On peut citer les genres Saccharomyces, tels que les *Saccharomyces boulardii* et *Saccharomyces cerevisiae* et les Kluyveromyces.

Les micro-organismes selon l'invention sont déshydratés. La taille desdits micro-organismes, après déshydratation, est généralement inférieure à 50 µm. Ils sont obtenus par les méthodes conventionnelles telles que le broyage après lyophilisation, par atomisation ou séchage en lit d'air fluidisé de biomasses plus ou moins concentrées et plus ou moins pures de micro-organismes.

En outre, les micro-organismes selon l'invention sont revivifiables. De ce fait, ils présentent la capacité de reprendre l'activité de la vie après une période d'anhydrobiose.

Les particules de la composition selon l'invention sont susceptibles de comprendre tous constituants, seuls ou en mélange, utilisés dans la fabrication ou la préparation d'une composition alimentaire ou médicamenteuse. Il s'agit en particulier de vitamines, d'oligo-éléments, de minéraux et de leurs sels, de charges telles que les sucres ou les aminoacides ou de farines végétales, seuls ou en mélange, apportant à ladite composition diverses propriétés, par exemple, physiques, telles que sa solubilisation en milieu aqueux ou une effervescence en présence d'un tel milieu, thérapeutiques, d'aide à la revivification, de complément nutritionnel ou d'appétibilité. Il peut aussi s'agir de principes actifs pharmaceutiques tels que, par exemple, des antibiotiques, des anti-inflammatoires, des anti-parasitaires, des antiacides, des analgésiques ou des tranquillisants. Les particules sont généralement substantiellement exemptes de micro-organismes.

A titre d'exemples non limitatifs de vitamines, on peut citer la vitamine A, la vitamine B1, la vitamine B2, la vitamine B3, la vitamine B4, la vitamine B5, la vitamine B6, la vitamine B8, la vitamine B9, la vitamine B12, la vitamine BT, la vitamine Bx, la vitamine C, la vitamine D, la vitamine D2, la vitamine D3, la vitamine E, la vitamine F, la vitamine G, la vitamine H, la vitamine K, la vitamine K3, la vitamine M, la vitamine P, la vitamine P4 et la vitamine PP. A titre d'exemples non limitatifs d'oligo-éléments, on peut citer le calcium, le cobalt, le cuivre, le fer, le fluor, l'iode, le magnésium, le manganèse, le sélénium et le zinc. A titre d'exemple non limitatifs de source de minéraux, on peut citer notamment le chlorure de calcium, le chlorure de potassium, le gluconate de cuivre, le fluorure de sodium, l'iodure de potassium, le sulfate de manganèse, le stéarate de magnésium, le sulfate de zinc et le succinate ferreux. A titre d'exemple non limitatif de charges, on peut citer les sucres et agents de charge tels que les fructanes, en particulier l'inuline, les fructo-oligosaccharides (FOS), les galacto-oligosaccharides (GOS), ou trans-galacto-oligosaccharides (TOS), les xylooligosaccharides (XOS), certaines maltodextrines, les polydextroses, le lactulose, les aminoacides et les fibres alimentaires. Les farines végétales sont par exemple des résidus de graines ou de fruits oléagineux, dont on a extrait l'huile.

Par ailleurs, les particules de la composition comportent avantageusement des agents effervescents formés d'un mélange d'au moins un acide et d'un agent alcalin. A titre d'exemples non limitatifs d'acides, on peut citer l'acide tartrique, l'acide citrique, l'acide maléique, l'acide fumarique, l'acide malique, l'acide adipique, l'acide succinique, l'acide lactique, l'acide glycolique, les alpha hydroxy acides, l'acide ascorbique et les acides aminés, ainsi que les sels et dérivés de ces acides. A titre d'exemples non limitatifs d'agents alcalins, on peut citer le bicarbonate de potassium, de sodium, le carbonate de potassium, de sodium, de calcium, d'ammonium ou le carbonate de L-lysine, d'arginine, de glycine sodique, les carbonates sodiques d'acides aminés.

Les constituants précités se présentent généralement sous une forme solide. Les particules sont sèches, au moins à leur surface, et se présentent bien souvent sous une forme sphérique, ovoïde ou allongée. Leur surface est lisse ou rugueuse et ce, de manière régulière ou irrégulière.

Selon l'invention, au moins 50% des particules ont un diamètre moyen supérieur à 250 µm et inférieur à 2000 µm, préférentiellement 1000 µm. Elles sont réparties dans un domaine de granulométrie pratiquement exclusivement compris entre 20 et 2000 µm et, préférentiellement, entre 50 et 1000 µm.

Les particules peuvent être obtenues par granulation et par suite se présenter sous la forme de granulés. Une telle granulation peut être effectuée par mélange en milieu humide, et comprendre des étapes de séchage puis de calibrage. Elle peut aussi être réalisée en lit d'air fluidisé ou sur des appareils connus de l'état de la technique combinant un ensemble d'opérations menant aux particules souhaitées, par compactage, par granulation ou par enrobage. Dans cette dernière alternative, les particules sont enrobées.

La composition peut comporter en outre des additifs alimentaires tel que des conservateurs, des anti-oxygènes, des supports, des acidifiants, des correcteurs d'acidité, des anti-agglomérants, des anti-moussants, des émulsifiants, des exhausteurs de goût, des agents moussants, des gélifiants, des agents d'enrobage, des humectants, des amidons modifiés, des séquestrants, des stabilisants, des épaississants et des agents de rétention d'eau.

La composition selon l'invention résulte par exemple d'un simple mélange mécanique de micro-organismes déshydratés et de particules tels que décrits ci-dessus. En pratique, elle comporte préférentiellement entre 1 et 80 % m/m (poids du poids total de la composition) de micro-organismes déshydratés et entre 20 et 99 % m/m de particules, et, plus préférentiellement, entre 10 et 40 % m/m de micro-organismes déshydratés et entre 60 et 90 % m/m de particules.

Par ailleurs, la composition selon l'invention est sèche, c'est-à-dire pas ou peu imprégnée de liquide et, en particulier d'eau. Elle présente préférentiellement une activité de l'eau inférieure à 0,3 et, plus préférentiellement, une activité de l'eau inférieure à 0,2.

La composition se présente sous une forme galénique quelconque normalement utilisée pour une administration par voie orale. Elle peut donc se présenter sous une forme pulvérulente et mise en sachets, pots ou sacs, ladite composition étant éventuellement effervescente, et destinée à être mise en solution dans de l'eau. Elle peut aussi se présenter sous la forme de capsules ou gélules, éventuellement gastro-résistantes, à enveloppe dure ou molle, ou alors sous forme de comprimés ou de granulés buccaux, sublinguaux ou effervescents, enrobés ou non, par exemple, à libération prolongée.

En pratique, les compositions selon l'invention peuvent être conservées pendant une période de temps relativement longue à l'abri de l'humidité et à température ambiante. De manière surprenante, la quantité de micro-organismes revivifiables dans ces compositions au bout de cette période de temps est largement supérieure à la quantité de micro-organismes revivifiables que l'on aurait pu obtenir dans des compositions de l'état de la technique, conservées dans de mêmes conditions. Ainsi, si l'on considère qu'à un temps t = 0 correspondant à son conditionnement, une composition comprend 100% de micro-organismes revivifiables, une telle composition comportera 18 mois après, si elle est conservée à l'abri de l'humidité et à température ambiante, après dénombrement du nombre de micro-organismes (par comptage des colonies obtenues après inoculation dans un ou sur un milieu de culture approprié) au moins environ 20% de micro-organismes revivifiables. Cela apparaît substantiel au regard des compositions selon l'état de la technique.

Dans un mode de réalisation préféré, la composition est soluble ou dispersible en milieu aqueux. Elle peut en plus être effervescente. Aussi, lorsqu'elle est ajoutée à de l'eau, par exemple destinée à l'alimentation des grands animaux tels que des équins ou des bovins, elle se dissout ou se disperse. L'effervescence facilite la dissolution ou la dispersion de la composition dans l'eau et sa diffusion dans ce milieu aqueux. Les micro-organismes revivifiables présents dans la composition sont réhydratés. La réhydratation est une étape rapide, qui dure quelques minutes. Au bout de ces quelques minutes, les grands animaux, absorbant l'eau dans laquelle la composition est dissoute ou dispersée, ingèrent des micro-organismes réhydratés ainsi que des composants utiles à leur organisme tels que des sucres, des vitamines ou des prébiotiques, si de tels éléments sont présents dans la composition de départ.

Il est cependant bien entendu que la composition est susceptible d'être utilisée à d'autres fins que pour l'alimentation des grands animaux. En effet, elle est susceptible de servir à l'alimentation de l'Homme et de tous animaux en particulier à sang chaud. Elle constitue un aliment ou un médicament à usage humain ou vétérinaire. Le choix d'un micro-organisme ou d'un mélange des micro-organismes est déterminé par l'utilisation finale de la composition selon l'invention.

Le procédé de préparation d'une composition alimentaire ou médicamenteuse selon l'invention est caractérisé en ce qu'il comporte les étapes de : - déshydratation de micro-organismes revivifiables; et de - mélange des micro-organismes déshydratés avec des particules dont au moins 50% ont un diamètre moyen supérieur à 250 µm.

La déshydratation est réalisée selon les méthodes conventionnelles décrites plus haut et le mélange avec des particules dont au moins 50% ont un diamètre moyen supérieur à 250 µm est effectué selon les techniques habituelles connues.

De manière préférée, le procédé de préparation est réalisé dans des conditions d'humidité contrôlées assurant une activité de l'eau inférieure à 0,3 dans la composition obtenue.

La présente invention va maintenant être illustrée au moyen des exemples suivants.

### Exemple 1

On prépare, de manière classique, par rotogranulation, des particules comportant des vitamines A, D3, E, K, B1, B2, B3, B5, B6, B12 et C, de l'acide folique, de la biotine, de la choline, de la lysine, de la méthionine, du tryptophane, de l'acide citrique, du carbonate et du bicarbonate de sodium, un absorbant et un émulsifiant. Le calibre des particules est ajusté de manière à ce que lesdites particules présentent un profil granulométrique compris entre 50 et 1000 µm, 65% desdites particules ayant une taille supérieure à 250 µm. On mélange ensuite mécaniquement 67% en poids du poids total (m/m) de telles particules avec 33% m/m d'un mélange de ferments comprenant les micro-organismes suivants : *Lactobacillus plantarum* et *Streptococcus infantarius*, titrant 2,52 x 10⁹ CFU, commercialisé par la société Lallemand™ sous l'appellation Adjulact^{™}. L'activité de l'eau de la composition effervescente A ainsi obtenue selon l'invention est de 0,17.

### Exemple 2

On prépare, de manière classique, par rotogranulation, des particules comportant de l'Enerlyte^{™} (commercialisé par la société Virbac^{™}) et, pour créer une effervescence, de l'acide citrique, du carbonate de sodium et du bicarbonate de sodium. Le calibre des particules est ajusté de manière à ce qu'elles présentent un profil granulométrique compris entre 50 et 1000 µm, 54% desdites particules ayant une taille supérieure à 250 µm. On mélange ensuite mécaniquement 67% m/m de telles particules avec 33% m/m d'un mélange de ferments comportant les bactéries suivantes *Lactobacillus plantarum*, *Streptococcus infantarius*, ledit mélange étant titré à 2,52 x 10⁹ CFU (Adjulact^{™} de Lallemand^{™}). On obtient alors une composition effervescente selon l'invention dont l'activité de l'eau du mélange est de 0,20.

### Exemple 3

On prépare un comprimé selon l'invention, contenant 3 x 10⁹ CFU de *Saccharomyces cerevisiae* lyophilisés, de formule :

| Constituants | Formule unitaire | Formule centésimale |
|---|---|---|
| *Saccharomyces cerevisiae* lyophilisés* | 499,94 mg | 35,71 |
| Mannitol | 689,22 mg | 49,23 |
| Crospovidone | 28,00 mg | 2 |
| Povidone | 56,00 mg | 4 |
| Cellulose microcristalline | 126,00 mg | 9 |
| Stéarate de magnésium | 0,84 | 0,06 |
| TOTAL | 1400,00 mg | 100 |
| Liquide de mouillage : | éliminé par séchage après granulation | |
| Eau | qs | |
| Ethanol | qs | |

| | | |
|---|---|---|
| ** Saccharomyces cerevisiae* lyophilisés titrant 6 x 10⁹ CFU/g | | |

On granule le mannitol, la crospovidone, la cellulose monocristalline avec la solution hydro-alcoolique de Povidone. Le granulé obtenu est séché et calibré pour avoir un granulé de taille inférieur à 800 micromètres. 82% des particules ont une taille supérieure à 250 micromètres.

On homogénéise les micro-organismes avec le granulé calibré obtenu dans un mélangeur à sec.

On procède à la compression avec une comprimeuse équipée de matrices et de poinçons de diamètre 17 mm ; on pulvérise préalablement sur les parois des matrices et sur les poinçons du stéarate de magnésium en tant que lubrifiant (la quantité excédentaire non adhérente de stéarate de magnésium étant aspirée avant la compression).

La force de compression est de l'ordre de 10 à 16 kN ce qui permet d'obtenir des comprimés conservant dans leur structure l'intégrité de la majorité des particules estimée à plus de 50%.

La quantité de stéarate de magnésium réparti à la surface d'un comprimé est de 0,84 mg soit 0,6 pour mille.

### Exemple 4

Une étude comparative de la viabilité des bactéries déshydratées *Lactobacillus plantarum et Streptococcus infantarius* présentes dans différentes compositions a été menée dans les conditions suivantes.

La composition A selon l'invention, décrite dans l'Exemple 1, a été répartie par 25 g, en sachets aluminisés soudés, qui offrent une très bonne barrière aux gaz et à l'humidité.

Par ailleurs, on a préparé une composition B, qui comprend exactement les mêmes bactéries et constituants des particules que la composition A, selon les mêmes quantités et proportions, mais dont les particules présentent, dans une large majorité, un diamètre moyen inférieur à 150 µm. De même que pour la composition A, cette composition B, qui se présente sous la forme d'une poudre, a été répartie par 25 g dans des sachets aluminisés soudés identiques à ceux de la composition A.

Enfin, on a préparé une composition C comprenant exactement les mêmes bactéries et constituants des particules que les compositions A et B selon le même procédé que celui mis en oeuvre dans l'exemple 1. Toutefois, dans ce cas, les micro-organismes sont parties constituantes des particules. Le diamètre moyen des particules de cette composition C est supérieur à 300 µm. Comme précédemment, cette composition C a été répartie par 25 g en sachets aluminisés soudés identiques à ceux des compositions A et B.

Puis, les différents sachets des compositions A, B et C ont été placés dans une étuve maintenue à une température de 40°C et en présence de 75% d'humidité. Un sachet de chacune des compositions a été analysé chaque semaine en vue de déterminer la teneur de chaque sachet en bactéries revivifiables. Les résultats sont reportés dans le tableau 1 ci-après. Ils sont exprimés en CFU/g et en pourcentage de la concentration initiale en micro-organismes revififiables :

**Tableau 1**

| | Temps | | | | | | |
|---|---|---|---|---|---|---|---|
| | 0 | 7 jours | 14 jours | 21 jours | 28 jours | 42 jours | 50 jours |
| Composition A | 4,18 x 10⁸ (100%) | 3,71 x 10⁸ (88,75%) | 3,05 x 10⁸ (72,85%) | 2,12 x 10⁸ (52,72%) | 1,10 x 10⁸ (26,20%) | 1,10 x 10⁸ (26,20%) | 1,10 x 10⁸ (26,20%), |
| Composition B | 2,52 x 10⁸ | 1,11 x 10⁸ | 1,32 x 10⁸ | 3,54 x 10⁷ | 0,21 x 10⁷ | - | - |
| | (100%) | (43,94%) | (52,18%) | (14,04%) | (0,8%) | (0%) | (0%) |
| Composition C | 2,94 x 10⁸ | 0,15 x 10⁸ | 0,32 x 10⁷ | - | - | - | - |
| | (100%) | (55%) | (1%) | (0%) | (0%) | (0%) | (0%) |

Il apparaît que la concentration initiale en bactéries viables de la composition A est supérieure à la concentration initiale en bactéries viables de la composition C qui est elle-même supérieure à la concentration initiale en bactéries de la composition B. En conséquence, il semble que les étapes de préparation des compositions B et C aient occasionné des stress plus importants aux bactéries que les étapes de préparation de la composition A.

Pour toutes les compositions, la concentration en bactéries revivifiables décroît en fonction du temps. Toutefois, cette concentration décroît moins lentement dans le cas de la composition A selon l'invention que dans le cas des autres compositions.

En outre, dans le cas de la composition A, la concentration en bactéries revivifiables décroît jusqu'à l'obtention d'une limite correspondant à environ 26,20% des 100% de bactéries revivifiables présentes initialement dans les sachets. En conséquence, la concentration en bactéries revivifiables apparaît stabilisée dans le temps, au moins jusqu'à 50 jours après la mise en sachet, dans les conditions opératoires précitées.

Bien sûr, cette stabilité constatée dans des conditions stressantes devrait, a *fortiori,* être constatée dans des conditions réelles de conservation de sachets à température ambiante. Compte tenu du fait qu'une semaine de conservation des sachets dans les conditions opératoires du présent exemple équivaut à environ 3 mois de conservation à 25 °C, il est permis de conclure qu'une composition selon l'invention telle que la composition A, conservée en sachets à des températures de l'ordre de 25°C, devrait comporter une quantité substantielle de bactéries revivifiables, en pratique environ 26,20% de la concentration initiale, 18 mois après la mise en sachets. A tout le moins, le présent exemple met en évidence une viabilité nettement améliorée des micro-organismes déshydratés par rapport à une composition substantiellement exempte de particules de diamètre moyen est bien inférieur à 250 µm (composition B) et par rapport à une composition dans lesquelles les micro-organismes sont parties constituantes des particules (composition C).

## Revendications

1. Composition alimentaire ou médicamenteuse sèche, comprenant entre 1 et 80% m/m de micro-organismes déshydratés revivifiables, **caractérisée en ce qu'**elle comprend en outre entre 20 et 99 % m/m de particules réparties dans un domaine de granulométrie compris entre 20 et 2000 µm dont au moins 50% ont un diamètre moyen supérieur à 250 µm, lesdites particules comprenant des constituants utilisés dans la fabrication ou la préparation d'une composition alimentaire ou médicamenteuse.

2. Composition selon la revendication 1, **caractérisée en ce que** les micro-organismes sont des bactéries ou des levures.

3. Composition selon la revendication 2, **caractérisée en ce que** les micro-organismes sont choisis parmi les familles des *Lactobacillaceae,* des *Bifidobacteriaceae,* des *Streptococcaceae,* des *Enterococcaceae,* des *Bacillaceae* ou des *Saccharomycetaceae,* seuls ou en mélanges.

4. Composition selon l'une des revendications 1, 2 ou 3, **caractérisée en ce qu'**elle comprend entre 10 et 40 % m/m, de micro-organismes.

5. Composition selon l'une des revendications précédentes, **caractérisée en ce que** les particules comportent des vitamines, des oligo-éléments, des aminoacides, des sucres, des minéraux ou leurs sels, des charges ou des farines végétales, seuls ou en mélange.

6. Composition selon l'une des revendications précédentes, **caractérisée en ce que** les particules comportent des agents effervescents.

7. Composition selon l'une des revendications précédentes, **caractérisée en ce qu'**elle comprend entre 60 et 90 % m/m de particules.

8. Composition selon l'une des revendications précédentes, **caractérisée en ce qu'**elle présente une activité de l'eau inférieure à 0,3.

9. Composition selon l'une des revendications précédentes, **caractérisée en ce qu'**elle se présente sous la forme de capsules ou de gélules, de comprimés ou de granulés.

10. Composition selon l'une des revendications précédentes, **caractérisée en ce qu'**elle est soluble ou dispersible dans l'eau.

11. Procédé de préparation d'une composition alimentaire ou médicamenteuse sèche, **caractérisé en ce qu'**elle comporte les étapes suivantes de :
- déshydratation de micro-organismes revivifiables; et de
- mélange des micro-organismes déshydratés avec des particules réparties dans un domaine de granulométrie compris entre 20 et 2000 µm dont au moins 50% ont un diamètre moyen supérieur à 250 µm.

12. Procédé selon la revendication 11, **caractérisé en ce qu'**il est réalisé dans des conditions d'humidité contrôlées assurant une activité de l'eau inférieure à 0,3 dans la composition obtenue.

13. Utilisation de la composition selon l'une des revendications 1 à 10 comme aliment ou pour la préparation d'un médicament, à usage humain ou vétérinaire.

## Claims

1. A dry food or drug composition, comprising between 1 and 80% m/m of dehydrated revivifiable microorganisms, **characterized in that** it further comprises between 20 and 99% m/m of particles having a particle size distribution in the range between 20 and 2000 µm, of which at least 50% have an average diameter of more than 250 µm, said particles comprising components used in the manufacture or preparation of a food or drug composition.

2. The composition according to claim 1, **characterized in that** the microorganisms are bacteria or yeasts.

3. The composition according to claim 2, **characterized in that** the microorganisms are chosen from the families consisting of *Lactobacillaceae, Bifidobacteriaceae, Streptococcaceae, Enterococcaceae, Saccharomycetaceae or Bacillaceae,* alone or in admixture.

4. The composition according to any one of claims 1, 2 or 3, **characterized in that** it comprises between 10 and 40% m/m of microorganisms.

5. The composition according to any one of the preceding claims, **characterized in that** the particles comprise vitamins, trace elements, amino acids, sugars, minerals or salts thereof, fillers or vegetable meals, alone or in admixture.

6. The composition according to any one of the preceding claims, **characterized in that** the particles comprise effervescent agents.

7. The composition according to any one of the preceding claims, **characterized in that** it comprises between 60 and 90% m/m of particles.

8. The composition according to any one of the preceding claims, **characterized in that** it has a water activity of less than 0.3.

9. The composition according to any one of the preceding claims, **characterized in that** it is in the form of capsules or hard gelatin capsules, tablets or granules.

10. The composition according to any one of the preceding claims, **characterized in that** it is soluble or dispersible in water.

11. A process for preparing a dry food or drug composition, **characterized in that** it comprises the steps of:
- dehydrating revivifiable microorganisms; and
- mixing dehydrated microorganisms with particles having a particle size distribution in the range between 20 and 2000 µm, of which at least 50% have an average diameter of more than 250 µm.

12. The method according to claim 11, **characterized in that** it is carried out under conditions of controlled humidity to ensure a water activity of less than 0.3 in the composition obtained.

13. Use of the composition according to any one of claims 1 to 10 as a food product or for the preparation of a drug, for human or veterinary use.

## Patentansprüche

1. Trockene Lebensmittel oder Medikament-Zusammensetzung, die zwischen 1 und 80% m/m dehydrierte wiederbelebbare Mikroorganismen beinhaltet, **dadurch gekennzeichnet, dass** sie zudem zwischen 20 und 99% m/m an Partikeln beinhaltet, die in einem Bereich der Korngrößenbestimmung zwischen 20 und 2000 µm verteilt sind, wovon mindestens 50% einen mittleren Durchmesser von mehr als 250 µm aufweisen, wobei die besagten Partikel Bestandteile beinhalten, die in der Herstellung oder Aufbereitung einer Zusammensetzung für Lebensmittel oder Medikamente verwendet werden.

2. Zusammensetzung nach Anspruch 1, **dadurch gekennzeichnet, dass** die Mikroorganismen Bakterien oder Hefearten sind.

3. Zusammensetzung nach Anspruch 2, **dadurch gekennzeichnet, dass** die Mikroorganismen einzeln oder vermischt aus den Familien der Lactobacillaceae, Bifidobacteriaceae, Streptococcaceae, Enterococcaceae, Bacillaceae oder Saccharomycetaceae, ausgewählt werden.

4. Zusammensetzung nach einem der Ansprüche 1, 2 oder 3, **dadurch gekennzeichnet, dass** sie Mikroorganismen zwischen 10 und 40% m/m beinhaltet.

5. Zusammensetzung nach einem der vorhergehenden Ansprüche, **dadurch gekennzeichnet, dass** die Partikel einzeln oder vermischt Vitamine, Spurenelemente, Aminosäuren, Zucker, Minerale oder deren Salze, pflanzliche Anteile oder pflanzliche Mehle enthalten.

6. Zusammensetzung nach einem der vorhergehenden Ansprüche, **dadurch gekennzeichnet, dass** die Partikel Brausemittel beinhalten.

7. Zusammensetzung nach einem der vorhergehenden Ansprüche, **dadurch gekennzeichnet, dass** zwischen 60 und 90% m/m an Partikeln beinhaltet.

8. Zusammensetzung nach einem der vorhergehenden Ansprüche, **dadurch gekennzeichnet, dass** sie eine Wasseraktivität unterhalb von 0,3 aufweist.

9. Zusammensetzung nach einem der vorhergehenden Ansprüche, **dadurch gekennzeichnet, dass** sie in Form von Kapseln oder Gel-Kapseln, Tabletten oder Granulaten vorliegt.

10. Zusammensetzung nach einem der vorhergehenden Ansprüche, **dadurch gekennzeichnet, dass** sie in Wasser löslich oder dispergierbar ist.

11. Verfahren zur Aufbereitung einer trockene Lebensmittel oder Medikament-Zusammensetzung, **dadurch gekennzeichnet, dass** es die folgenden Schritten umfasst:
- Dehydrieren der wiederbelebbaren Mikroorganismen; und
- Vermischung der dehydrierten Mikroorganismen mit Partikeln, die in einem Bereich der Korngrößenbestimmung zwischen 20 und 2000 µm verteilt sind, wovon mindestens 50% einen mittleren Durchmesser von mehr als 250 µm aufweisen.

12. Verfahren nach Anspruch 11, **dadurch gekennzeichnet, dass** es unter kontrollierten Feuchtigkeitsbedingungen durchgeführt wird, die in der erhaltenen Zusammensetzung eine Wasseraktivität von weniger als 0,3 sicherstellen.

13. Verwendung der Zusammensetzung nach einem der Ansprüche 1 bis 10 als Lebensmittel oder zur Aufbereitung eines Medikaments, zur humanen oder veterinären Anwendung.
